# EUROPEAN PATENT APPLICATION

(11) **EP 1 378 256 A1**
(43) Date of publication of application: **07.01.2004**
(21) Application number: 02708637.0
(22) Date of filing: 22.03.2002
(51) Int. Cl.: A61L 27/00, A61F 2/28

(54) **ARTIFICIAL BONE MATERIAL**

(30) Priority: 23.03.2001 JP 2001084525
(71) Applicant: Olympus Optical Co., Ltd., Tokyo 151-0072 (JP); National Institute of Advanced Industrial Science and Technology, Tokyo 100-0013 (JP)
(72) Inventor: HAKAMAZUKA, Yasuharu, Olympus Corporation, Shibuya-ku, Tokyo 151-0072 (JP); IRIE, Hiroyuki, Olympus Corporation, Shibuya-ku, Tokyo 151-0072 (JP); INOUE, Hikaru, Olympus Corporation, Shibuya-ku, Tokyo 151-0072 (JP); MASUBUCHI, Ryouji, Olympus Corporation, Shibuya-ku, Tokyo 151-0072 (JP); OKABE, Hiroshi, Olympus Corporation, Shibuya-ku, Tokyo 151-0072 (JP); UEMURA, Toshimasa, Tsukuba-shi, Ibaraki 305-0044 (JP)
(74) Representative: von Hellfeld, Axel, Dr. Dipl.-Phys.
(86) International application number: PCT/JP2002/002744
(87) International publication number: WO 2002/076522

(57) **Abstract**

An artificial bone material having a satisfactory compatibility with a human body and capable of effecting osteogenesis satisfactorily which is obtained by integrating a marrow cell in a porous ceramic consisting of β-tricalcium phosphate.

## Description

### TECHNICAL FIELD

The present invention relates to an artificial bone material employed for repairing bone defects.

### BACKGROUND ART

Recently, artificial bones have been increasingly employed in the field of, for example, orthopedics for repairing bone defects caused by various diseases. The artificial bone is generally made of calcium phosphate-based ceramic. This ceramic is highly biocompatible, has satisfactory bone conductivity, and acts as a foothold for osteogenesis. However, the calcium phosphate-based ceramic when employed alone cannot serve to repair a highly severe bone defect. Accordingly, the only option an autograft implantation, and hence it is difficult to repair the bone defect when the amount of the bone to be collected is limited or in some other situations.

Under such a circumstance, an implantation material which has a further higher osteogenetic ability, i.e., has a bone-inducing activity, is demanded in a case where severity of the bone defect is high. To respond to such a demand, a cell-incorporated artificial bone obtained by incubating a marrow cell using a calcium phosphate-based ceramic material described above as a carrier has been investigated.

Yoshikawa et al. observed significant osteogenesis when they mixed cultured human marrow cells with porous hydroxyapatite (HAP), incubated the hydroxyapatite in an osteogenetic medium for 3 weeks, implanted the incubated hydroxyapatite into an abdominal cavity of a nude mouse, extracted the hydroxyapatite after 2 months, and then made histological evaluation of the hydroxyapatite (J. Jpn. Orthop. Assoc., 73 (3), S672).

An artificial bone obtained by incorporating cultured marrow cells into a porous ceramic is associated with the following problems.

Firstly, the introduction of marrow cells into a central portion of a porous ceramic makes it difficult-for the marrow cell to enter the central portion of the ceramic when the porous ceramic used has a large size. In addition, even when the cell is introduced into the central portion of the porous ceramic, the cell cannot serve as an osteoblast under a reduced partial pressure of oxygen resulting from the absence of enough blood vessels reaching the central portion of the porous ceramic.

Secondly, a porous material as a carrier for incorporating a cell has the following problems. A material serving as a carrier should fulfill the following conditions. While it is a matter of course that the carrier should be highly biocompatible and should not interfere with the activity of the cultured cells, it is important that the carrier should have a bone conductivity and after implantation, the implanted carrier itself should be absorbed gradually as the osteogenesis proceeds. While collagen or polylactate glycolate as a carrier is biodegradable and satisfactory with regard to the decomposition and absorption performances out of the requirements for a carrier, collagen or polylactate glycolate has a poor bone conductivity and is undesirable in this respect.

On the other hand, a calcium phosphate-based ceramic is excellent in the bone conductivity, and it is preferable in this respect. Nevertheless, an HAP, which is most common as an artificial bone among calcium phosphates, is not preferable in respect of the absorption performance because of its poor in vivo absorption behavior. On the contrary, β-tricalcium phosphate (β-TCP) exhibits a satisfactory absorption performance. Taking this into consideration in combination with its bone conduction performance, β-TCP has been considered to be the most preferable material as a carrier.

From such a viewpoint, β-TCP has been employed alone as a bone prosthetic material. However, Altermatt et al. reported in Eur. J. Pediatr. Surg., 2, 180-182 reported that when applied to a bone cyst and subjected to a follow-up observation, porous β-TCP still remained in the prosthetic site even after a period as long as 7 years. While β-TCP naturally has an ability to be absorbed, it sometimes remains for such a long period, suggesting that it is not always sufficient that β-TCP is used.

Practically, the purity of β-TCP should be considered. β-TCP is produced generally by a dry process in which calcium carbonate and calcium hydrogen phosphate are subjected to a solid phase reaction or by a wet process in which a calcium (Ca) ion and a phosphate (P) ion are reacted.

In the dry process, however, some unreacted substances may remain or a resultant powder has a poor sintering performance since the reaction proceeds non-uniformly. In the wet process, the temperature and pH should be adjusted precisely, and in some cases the ratio between Ca to P may be slightly deviated from the stoichiometric value and the product may contain a small amount of by-products. The characteristics of a material largely depend on the process in which it is produced, and a poor process leads to a failure in obtaining desired results in the stages of application and practical use, and no study in this respect has been made so far. Moreover, the state of the pores of porous β-TCP serves also as a factor which exerts an influence on the bone conductivity and the absorption performance.

It is an object of the present invention to provide an ideal artificial bone material which promotes osteogenesis by combining β-TCP with a cultured marrow cell.

### DISCLOSURE OF THE INVENTION

An artificial bone material according to claim 1 includes a porous ceramic consisting essentially of β-tricalcium phosphate, and a marrow cell incorporated in the porous ceramic.

The invention of claim 2 is an artificial bone material according to claim 1, wherein the marrow cell is further combined with a cell growth factor contributing to osteogenesis.

The invention of claim 3 is an artificial bone material according to claim 1, wherein the porous ceramic has a porosity of 60% to 90% and includes macropore of size 50 µm to 1,000 µm that communicate to each other and micropores of size 2 µm or less that communicate to each other.

The invention of claim 4 is an artificial bone material according to claim 1 or 3, wherein the porous ceramic is one produced by molding a β-tricalcium phosphate powder synthesized by a mechanochemical method as a raw material, and then sintering the resultant.

The invention of claim 5 is an artificial bone material according to claim 1 or 2, wherein the marrow cell is a cultured cell collected from a patient and incubated.

The invention of claim 6 is an artificial bone material according to claim 5, wherein the cultured cell is one subjected to at least one selected from the group consisting of electric stimulation and mechanical stimulation during incubation.

The invention of claim 7 is an artificial bone material according to claim 5 or 6, wherein the cultured cell is one inoculated into an inside of the porous ceramic by at least one of or a combination of (a) to (c):
(a) inoculating the cultured cell under reduced pressure or increased pressure;
(b) inoculating the cultured cell with reducing and increasing the pressure alternatingly; and,
(c) inoculating the cultured cell with exerting a centrifugal force.

### BEST MODE FOR CARRYING OUT THE INVENTION

The artificial bone material according to the present invention includes a porous ceramic consisting of β-TCP and a marrow cell incorporated by inoculation in the porous ceramic. The morphology of the porous ceramic may be in the form of, for example, a block or granule.

In order to ensure the introduction of cells into the central portion of the porous ceramic, the cultured marrow cells are inoculated in the porous ceramic under reduced pressure or increased pressure, with reducing and increasing the pressure alternatingly or with exerting a centrifugal force. In such a manner, the marrow cells can be introduced into the central portion of the porous ceramic. In this procedure, it is effective to use a plurality of such means in combination.

In addition, at least one selected from the group consisting of electric stimulation or mechanical stimulation such as application of electric field, isotropic pressure or shock wave during the incubation of cells increases the cell growth rate, so that the activity of cells is maintained.

The vascularization in the porous ceramic becomes possible by combining an inducing factor that contributes to the vascularization, such as VEGF, with a marrow cell. In such a case, it is preferable to combine the inducing factor by gene transduction using a VEGF expression vector.

Also, combining in addition to the cultured cell, not only VEGF but also a cell growth factor that contributes to osteogenesis can accomplish a more preferable osteogenesis. For example, cell growth factors that contribute to osteogenesis, such as BMP, FGF, TGF-β, IGF and PDGF, can be employed to ensure the osteogenesis.

An artificial bone material in which a porous ceramic consisting of β-TCP is combined with a cultured marrow cell can promote osteogenesis. The β-TCP in this artificial bone material is one having a high purity and excellent bone conductivity and absorption performance.

The porous ceramic consisting of β-TCP, includes macropores that communicate to each other and micropores that communicate to each other and are smaller than the macropores, and the porous ceramic has a porosity of preferably 60% to 90%. The size of the macropores is preferably 50 µm to 1,000 µm, more preferably 100 µm to 500 µm. The macropores are present in an amount of preferably about 30% to 70% based on the void volume ratio of the entire pores. The macropores contribute to the introduction of marrow cells in the ceramic and to vascularization.

The micropores have a size of preferably 0.2 µm or less, more preferably 0.1 µm or less. The micropores are present in an amount of preferably about 10% to 40% based on the void volume ratio of the entire pores. The micropores contribute to promoting a chemical effect such as susceptibility to absorption.

As a highly pure β-TCP, one produced by a mechanochemical method, which is a wet process pulverization method, is excellent as a component of a material employed as a prosthetic material of a bone tissue. In this mechanochemical method, calcium carbonate and calcium hydrogen phosphate dihydrate are weighed in such amounts that the molar ratio of Ca to P is 1.5 and these powder are subjected to a wet pulverization method using a ball mill to obtain a slurry which is then dried and sintered at 720°C to 900°C to obtain β-TCP. By this method, the ratio of Ca to P can be controlled on the basis of the weighed amount of the raw material, and β-TCP having a high purity and an excellent sintering performance can be obtained.

A porous ceramic consisting of β-TCP having excellent bone conductivity and absorption performance is produced as described below. To a β-TCP powder obtained by a wet pulverization method is added a surfactant (deflocculating agent) and molded as a wet foam, which is then dried and sintered at 950°C to 1,050°C to form a porous article. By this method, a porous ceramic which has a porosity of 60% to 90% and includes macropores of size 50 µm to 1,000 µm consisting of plural pores communicating to each other at a void volume ratio of 30% to 70% based on the entire pores and micropores consisting of plural proes communicating to each other of size 2 µm or less at a void volume ratio of 10% to 40% based on the entire pores can be obtained.

By combining a porous ceramic consisting of β-TCP and a marrow cell to form an artificial bone material as described above, an artificial bone material that can promote osteogenesis satisfactorily can be obtained.

### (Example 1)

A calcium carbonate powder and calcium hydrogen phosphate dihydrate were weighed in the molar ratio of 1:2, and placed in a ball mill pot together with pure water, and mixed and pulverized for about one day using a ball mill. The resultant slurry was dried at about 80°C, and then sintered at 750°C. The resultant powder was a highly pure β- TCP ceramic having an excellent sintering performance.

To the powder were added pure water, an ammonium acrylate-based deflocculating agent, and a polyoxyethylene alkylphenyl ether-based surfactant, and then the resultant was mixed and stirred to obtain a foamed slurry. This foamed slurry was dried and then sintered at 1,050°C to obtain a β-TCP porous ceramic. The porous ceramic had a porosity of 75%, and a pore size distributed within two ranges, namely, 100 mµ to 500 mµ and 1 mµ to 0.1 mµ.

### (Second Example 2)

Using the β-TCP porous ceramic produced in Example 1 as a foothold material, marrow-derived osteoblast-like primary culture cells were inoculated and subjected to in vitro culture to form a bone tissue serving as a seed for osteogenesis. The bone tissue was implanted into a living body, and then the implanted tissue was allowed to form a large amount of a bone tissue. Specific procedure is described as follows.

Marrow cells were taken, transferred into a culture flask, to which an MEM medium supplemented with 10% to 15% FBS (fetal bovine serum) was added, and incubated for about 10 days under a 5% CO₂ atmosphere at 37°C. Subsequently, the cells were peeled off from the culture flask by a trypsin treatment, and then inoculated to a porous ceramic consisting of β-TCP in the form of a block. As a medium, an MEM medium containing 10 to 15% FBS (fetal bovine serum) was employed.

The cell density for the inoculation to a block of 5 mm × 5 mm × 5 mm was required to be 1,000,000 cells or more per cubic centimeter of medium. After incubation for 1 hours to 3 hours under a 5% CO₂ atmosphere at 37°C, the medium was exchanged with an MEM medium containing 10% to 51% FBS (fetal bovine serum) as a base medium to which 10⁻⁸ M dexamethasone, 10 mM β-glycerophosphate, and 50µg/ml ascorbic acid had further been supplemented, and then the incubation was conducted for about 2 weeks under a 5% CO₂ atmosphere at 37°C with exchanging the medium at intervals of 2 days. Subsequently, the cells together with the block were implanted into a living body.

Thereafter, a bone marrow fluid taken from a thigh bone of a Fisher rat was incubated as described above and inoculated onto a block consisting of a β-TCP porous ceramic. The inoculated porous ceramic was incubated for 2 weeks, implanted subcutaneously into another Fisher rat, and isolated after 3 weeks. The isolated implant was examined by HE staining, which revealed satisfactory osteogenesis.

### (Example 3)

A cell growth factor was adsorbed onto the β-TCP porous ceramic produced in Example 1 to effect inoculation. As the cell growth factor, each of VEGF, BMP, FGF, FGF-β, IGF and PDGF was employed. Then an in vitro culture was performed to form a bone tissue serving as a seed for osteogenesis. The bone tissue was implanted into a living body, and then the implanted tissue was allowed to form a large amount of a bone tissue. Specific procedure is described as follows.

Each cell growth factor described above was taken, transferred into a culture flask, to which an MEM medium supplemented with 10% to 15% FBS (fetal bovine serum) was added, and incubated for about 10 days under a 5% CO₂ atmosphere at 37°C. Subsequently, the cells were peeled off from the culture flask by a trypsin treatment, and then inoculated to a porous ceramic consisting of β-TCP in the form of a block. As a medium, an MEM medium containing 10% to 15% FBS (fetal bovine serum) was employed.

For the inoculation, techniques in which each cell growth factor was inoculated under reduced pressure, inoculated under increased pressure and inoculated with reducing and increasing the pressure alternatingly were conducted, respectively, together with a method in which a centrifugal force was applied, and samples under respective inoculation conditions were prepared.

The concentration of a cell growth factor for the inoculation to a block of 5 mm × 5 mm × 5 mm was required to be 1,000,000 cells or more per cubic centimeter of medium. After incubation for 1 to 3 hours under a 5% CO₂ atmosphere at 37°C, the medium was exchanged with an MEM medium containing 10% to 15% FBS (fetal bovine serum) as a base medium to which 10⁻⁸ M dexamethasone, 10 mM β-glycerophosphate, and 50 µg/ml ascorbic acid had further been supplemented, and then the incubation was conducted for about 2 weeks under a 5% CO₂ atmosphere at 37°C with exchanging the medium at intervals of 2 days. Subsequently, the cells together with the block were implanted into a ,living body.

Thereafter, a bone marrow fluid taken from a thigh bone of a Fisher rat was incubated as described above, inoculated onto a block consisting of a β-TCP porous ceramics, incubated for 2 weeks. The block was implanted subcutaneously into another Fisher rat, and isolated after 3 weeks. The isolated implant was examined by HE staining, which revealed satisfactory osteogenesis.

### INDUSTRIAL APPLICABILITY

As described above, the present invention provides an artificial bone material that can promote osteogenesis satisfactorily by incorporating a marrow cell into a β-TCP porous ceramic. Also, by combining with a mechanical stimulation such as isotropic pressure or with a cell growth factor such as VEGF, the osteogenesis can further be ensured, resulting in an enhanced usefulness.

## Claims

1. An artificial bone material, comprising a porous ceramic consisting of β-tricalcium phosphate and a marrow cell incorporated in the porous ceramic.

2. The artificial bone material according to claim 1, further comprising a cell growth factor that contributes to osteogenesis, combined with the marrow cell.

3. The artificial bone material according to claim 1, wherein the porous ceramic has a porosity of 60% to 90% and includes macropores of size 50 µm to 1,000 µm that communicate to each other and micropores of size 2 µm or less that communicate to each other.

4. The artificial bone material according to claim 1 or 3, wherein the porous ceramic is produced by molding a β-tricalcium phosphate powder synthesized by a mechanochemical method as a raw material, and then sintering the resultant.

5. The artificial bone material according to claim 1 or 2, wherein the marrow cell is a cultured cell collected from a patient and incubated.

6. The artificial bone material according to claim 5, wherein the marrow cell is subjected to at least one selected from the group consisting of electric stimulation and mechanical stimulation during incubation.

7. The artificial bone material according to claim 5 or 6, wherein the marrow cell is inoculated in the porous ceramic by means of at least one of or a combination of (a) to (c):
(a) inoculating the cultured cell under reduced pressure or increased pressure;
(b) inoculating the cultured cell with reducing and increasing the pressure alternatingly; and,
(c) inoculating the cultured. cell with exerting a centrifugal force.
